# EUROPEAN PATENT APPLICATION

(11) **EP 4 332 113 A1**
(43) Date of publication of application: **06.03.2024**
(21) Application number: 22861560.5
(22) Date of filing: 22.07.2022
(51) Int. Cl.: C07K 14/47, C12N 15/77, C12P 13/08

(54) **NOVEL REGULATOR OF ACETATE METABOLISM A-VARIANT, AND METHOD FOR PRODUCING L-BRANCHED CHAIN AMINO ACID USING SAME**

(30) Priority: 27.08.2021 KR 20210114069
(71) Applicant: CJ Cheiljedang Corporation, Seoul 04560 (KR)
(72) Inventor: LEE, Heeseok, Seoul 04560 (KR); KIM, Seon Hye, Seoul 04560 (KR); CHOI, Sun Hyoung, Seoul 04560 (KR); KIM, Ju-yeon, Seoul 04560 (KR); YOON, Byoung Hoon, Seoul 04560 (KR); SHIM, Jihyun, Seoul 04560 (KR); OH, Haena, Seoul 04560 (KR); YUN, Hyojin, Seoul 04560 (KR)
(74) Representative: Regimbeau
(86) International application number: PCT/KR2022/010784
(87) International publication number: WO 2023/027348

(57) **Abstract**

Provided are a novel variant regulator of acetate metabolism A, a polynucleotide encoding the variant, a microorganism for producing L-branched-chain amino acids, the microorganism including the variant or the polynucleotide, and a method of producing L-branched-chain amino acids using the microorganism.

## Description

### BACKGROUND OF THE INVENTION

### 1. Field of the Invention

The present disclosure relates to a novel variant regulator of acetate metabolism A, a polynucleotide encoding the variant, a microorganism for producing L-branched-chain amino acids, the microorganism including the variant or the polynucleotide, and a method of producing L-branched-chain amino acids using the microorganism.

### 2. Description of the Related Art

L-Amino acids are basic structural units of proteins and are used as an important raw material for pharmaceutical raw materials, food additives, animal feeds, nutrients, insecticides, bactericides, *etc.* In particular, branched-chain amino acid (BCAA) is a general term for L-valine, L-leucine, and L-isoleucine, which are essential amino acids. The branched-chain amino acids are known to have antioxidant effects and the effect of directly promoting protein synthesis in muscle cells.

Meanwhile, production of branched-chain amino acids using microorganisms is mainly carried out through microorganisms of the genus *Escherichia* or microorganisms of the genus *Corynebacterium,* and branched-chain amino acids are known to be biosynthesized using 2-ketoisocaproate as a precursor from pyruvic acid through several steps (Korean Patent No. 10-0220018, Korean Patent No. 10-0438146). However, the production of L-branched-chain amino acids using microorganisms has a problem in that industrial mass production is not easy.

With this background, the present inventors have found that the production ability of L-branched-chain amino acids may be remarkably increased when a variant with enhanced activity of a regulator of acetate metabolism A of a microorganism (hereinafter referred to as RamA, J Bacteriol. 2006 Apr;188(7):2554-67., Applied Microbiology and Biotechnology (2018) 102:5901-5910) is introduced for the purpose of improving the production ability of L-branched-chain amino acids by microorganisms.

The present inventors have developed a novel variant regulator of acetate metabolism A, which improves the production of L-branched-chain amino acids, a polynucleotide encoding the variant, a microorganism for producing L-branched-chain amino acids, the microorganism including the variant or the polynucleotide, and a method of producing L-branched-chain amino acids using the microorganism, thereby completing the present disclosure.

### SUMMARY OF THE INVENTION

An object of the present disclosure is to provide a variant regulator of acetate metabolism A, in which an amino acid corresponding to position 56 in an amino acid sequence of SEQ ID NO: 3 is substituted with another amino acid.

Another object of the present disclosure is to provide a polynucleotide encoding the variant of the present disclosure.

Still another object of the present disclosure is to provide a microorganism for producing L-branched-chain amino acids, the microorganism including the variant of the present disclosure or the polynucleotide encoding the variant.

Still another object of the present disclosure is to provide a method of producing L-branched-chain amino acids, the method including the step of culturing the microorganism in a medium.

Still another object of the present disclosure is to provide a composition for producing L-branched-chain amino acids, the composition including the variant of the present disclosure, the polynucleotide encoding the variant, a vector including the polynucleotide, or the microorganism including the polynucleotide of the present disclosure; a medium in which the microorganism is cultured; or a combination of two or more thereof.

Still another object of the present disclosure is to provide use of the variant regulator of acetate metabolism A, in which the amino acid corresponding to position 56 in the amino acid sequence of SEQ ID NO: 3 is substituted with another amino acid, in the production of L-branched-chain amino acids.

### DETAILED DESCRIPTION OF THE PREFERRED EMBODIMENTS

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a variant regulator of acetate metabolism A, in which an amino acid corresponding to position 56 in an amino acid sequence of SEQ ID NO: 3 is substituted with another amino acid.

The variant of the present disclosure may be those having enhanced activity by substituting an amino acid at a specific site in an amino acid sequence of the existing regulator of acetate metabolism A, but is not limited thereto.

In one embodiment, the variant regulator of acetate metabolism A may be a variant regulator of acetate metabolism A including one or more, or two or more amino acid substitutions in the amino acid sequence of SEQ ID NO: 3, but is not limited thereto.

Specifically, with regard to the variant of the present disclosure, the amino acid(s) corresponding to position 56 and/or position 52 in SEQ ID NO: 3 may be substituted with another amino acid, but is not limited thereto. More specifically, with regard to the variant, amino acids at any one or more of the positions or at both the positions or at positions corresponding thereto may be substituted with another amino acid, but is not limited thereto.

The "other amino acid" is not limited, as long as it is different from the amino acid before substitution. For example, when described as "substituting the amino acid corresponding to position 56 in SEQ ID NO: 3 with another amino acid", it means substitution with phenylalanine, glycine, alanine, arginine, aspartate, cysteine, glutamic acid (glutamate), asparagine, glutamine, histidine, proline, serine, tyrosine, isoleucine, lysine, tryptophan, valine, methionine, or threonine, excluding leucine, and when described as "substituting the amino acid corresponding to position 52 in SEQ ID NO: 3 with another amino acid", it means substitution with asparagine, glycine, arginine, aspartate, cysteine, glutamic acid, glutamine, histidine, proline, serine, tyrosine, isoleucine, leucine, lysine, phenylalanine, tryptophan, valine, methionine, or threonine, excluding alanine, but are not limited thereto.

Meanwhile, those skilled in the art may identify the amino acids corresponding to positions 56 and 52 of SEQ ID NO: 3 of the present disclosure in any amino acid sequence through sequence alignment known in the art, and even though otherwise specified in the present disclosure, it is apparent that description of "an amino acid at a specific position in a specific sequence number" means to include "an amino acid at a position corresponding thereto" in an arbitrary amino acid sequence. Therefore, an amino acid sequence, in which any one or more amino acids selected from the group consisting of the amino acids corresponding to positions 56 and 52 of SEQ ID NO: 3 are substituted with another amino acid, is also included in the scope of the present disclosure.

For example, when one or more, or two or more amino acids among amino acids corresponding to positions 56 and 52 of SEQ ID NO: 3 are substituted with another amino acid, it is possible to provide a variant having higher activity than the unsubstituted (unmodified) amin acid sequence.

Specifically, the variant of the present disclosure may be a variant in which the amino acids corresponding to positions 56 and 52 of SEQ ID NO: 3 are substituted with another amino acid, but is not limited thereto.

For specific example, the variant of the present disclosure may be a variant in which leucine, which is the amino acid corresponding to positions 56 of SEQ ID NO: 3, is substituted with alanine, and alanine, which is the amino acid corresponding to positions 52 of SEQ ID NO: 3, is substituted with valine, but is not limited thereto.

For more specific example, the variant of the present disclosure may have an amino acid sequence represented by SEQ ID NO: 1 or SEQ ID NO: 5, or may essentially consist of the amino acid sequence.

Further, the variant of the present disclosure may include substitution of amino acid(s) corresponding to positions 56 and/or 52 from the N-terminus of SEQ ID NO: 3 with another amino acids in an amino acid sequence having at least 70%, 75%, 80%, 85%, 90%, 95%, 96%, 97%, 98%, 99%, 99.5%, 99.7%, or 99.9% or more homology or identity to the amino acid sequence represented by SEQ ID NO: 1 or SEQ ID NO: 5. It is also apparent that a variant having an amino acid sequence having deletion, modification, substitution, conservative substitution, or addition of some amino acids also falls within the scope of the present disclosure as long as the amino acid sequence has such a homology or identity and exhibits efficacy corresponding to that of the variant of the present disclosure.

Examples thereof include those having sequence addition or deletion that does not alter the function of the variant of the present disclosure at the N-terminus, C-terminus of the amino acid sequence, and/or inside the amino acid sequence, naturally occurring mutation, silent mutation, or conservative substitution.

As used herein, the term "conservative substitution" means substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. For example, positively charged (basic) amino acids include arginine, lysine, and histidine; negatively charged (acidic) amino acids include glutamic acid and aspartate; aromatic amino acids include phenylalanine, tryptophan, and tyrosine, hydrophobic amino acids include alanine, valine, isoleucine, leucine, methionine, phenylalanine, tyrosine, and tryptophan. Further, amino acids may be divided into amino acids having electrically charged side-chain and amino acids having uncharged side-chain. The amino acids having electrically charged side-chain include aspartic acid, glutamic acid, lysine, arginine, histidine, and the amino acids having uncharged side-chain may also be divided into non-polar amino acids or polar amino acids. The non-polar amino acids my include glycine, alanine, valine, leucine, isoleucine, methionine, phenylalanine, tryptophan, and proline, and the polar amino acids may include serine, threonine, cysteine, tyrosine, asparagine, and glutamine. Usually, conservative substitution may hardly affect or not affect the activity of the produced polypeptides. Usually, conservative substitution may hardly affect or not affect the activity of proteins or polypeptides.

Further, the variant may include deletions or additions of amino acids that have minimal effect on the properties and secondary structure of the polypeptide. For example, the polypeptide may be conjugated with a protein N-terminal signal (or leader) sequence that is co-translationally or post-translationally involved in the protein translocation. Further, the polypeptide may be conjugated with other sequences or linkers so as to be identified, purified, or synthesized.

As used herein, the term "variant" refers to a polypeptide which has an amino acid sequence different from that of the variant before being varied by conservative substitution and/or modification of one or more amino acids but maintains the functions or properties. Such a variant may be generally identified by modifying one or more amino acids of the amino acid sequence of the polypeptide and evaluating the properties of the modified polypeptide. In other words, ability of the variant may be increased, unchanged, or decreased, as compared to that of the polypeptide before being varied. Further, some variants may include variants in which one or more portions such as an N-terminal leader sequence or a transmembrane domain have been removed. Other variants may include variants in which a portion of the N- and/or C-terminus has been removed from the mature protein. The term "variant" may be used interchangeably with terms such as modification, modified polypeptide, modified protein, mutant, mutein, and divergent, and is not limited thereto as long as it is a term used with the meaning of variation. With respect to the objects of the present disclosure, the variant may be a polypeptide including an amino acid sequence represented by SEQ ID NO: 1, in which alanine is substituted for leucine which is the amino acid corresponding to position 56 of SEQ ID NO: 3; or a polypeptide including an amino acid sequence represented by SEQ ID NO: 5, in which alanine is substituted for leucine which is the amino acid corresponding to position 56 of SEQ ID NO: 3 and valine is substituted for alanine which is the amino acid corresponding to position 52 of SEQ ID NO: 3.

Further, the variant may include deletions or additions of amino acids that have minimal effect on the properties and secondary structure of the polypeptide. For example, a signal (or leader) sequence that is co-translationally or post-translationally involved in the protein translocation may be conjugated to the N-terminus of the variant. The variant may be conjugated with other sequences or linkers so as to be identified, purified, or synthesized.

As used herein, the term "homology" or "identity" means the degree of similarity between two given amino acid sequences or base sequences, and may be expressed as a percentage. The terms "homology and identity" may often be used interchangeably.

The sequence homology or identity of a conserved polynucleotide or polypeptide is determined by standard alignment algorithms, and the default gap penalty established by a program used may be used together. Substantially, homologous or identical sequences are generally capable of being hybridized with the entirety or part of the sequence under moderately or highly stringent conditions. It is apparent that hybridization also includes hybridization with a polynucleotide including a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al. (1988) Proc. Natl. Acad. Sci. USA 85:2444. Alternatively, the homology, similarity, or identity may be determined using Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J., et al., Nucleic Acids Research 12:387 (1984)), BLASTP, BLASTN, FASTA (Atschul, S. F., ETAL, J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, BLAST of the National Center for Biotechnology Information or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443 as announced in, for example, Smith and Waterman, Adv. Appl. Math (1981) 2:482. In summary, the GAP program may be defined as the value acquired by dividing the number of similarly aligned symbols (*i.e.*, nucleotides or amino acids) by the total number of symbols in the shorter of two sequences. The default parameters for the GAP program may include (1) a binary comparison matrix (including values of 1 for identity and 0 for non-identity) and a weighted comparison matrix of Gribskov et al. (1986) Nucl. Acids Res. 14:6745 (or EDNAFULL (EMBOSS version of NCBI NUC4.4) substitution matrix) as disclosed in Schwartz and Dayhoff, eds., Atlas Of Protein Sequence And Structure, National Biomedical Research Foundation, pp. 353-358(1979); (2) a penalty of 3.0 for each gap and an additional 0.10 penalty for each symbol in each gap (or gap opening penalty of 10, gap extension penalty of 0.5); and (3) no penalty for end gaps.

For one example of the present disclosure, the variant of the present disclosure may have activity of the regulator of acetate metabolism A. Further, the variant of the present disclosure may have activity to increase the L-branched-chain amino acid producing ability, as compared to a wild-type polypeptide having the activity of the regulator of acetate metabolism A.

As used herein, the term "regulator of acetate metabolism A" is a target protein of the present disclosure, and refers to a regulatory protein related to acetate metabolism, and may be encoded by ramA gene.

In the present disclosure, expression of the regulator of acetate metabolism A may be enhanced, leading to an increase in the productivity of L-branched-chain amino acids.

As used herein, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, the "corresponding region" generally refers to a similar or corresponding position in a related protein or a reference protein.

For example, an arbitrary amino acid sequence is aligned with SEQ ID NO: 3, and based on this, each amino acid residue of the amino acid sequence may be numbered with reference to the numerical position of the amino acid residue corresponding to the amino acid residue of SEQ ID NO: 3. For example, a sequence alignment algorithm as described in the present disclosure may determine the position of an amino acid or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48:443-453), the Needleman program of the EMBOSS package (EMBOSS: The European Molecular Biology Open Software Suite, Rice et al., 2000), Trends Genet. 16:276-277), *etc.* may be used, but are not limited thereto, and a sequence alignment program, a pairwise sequence comparison algorithm, *etc.,* which known in the art, may be appropriately used.

Another aspect of the present disclosure provides a polynucleotide encoding the variant of the present disclosure.

As used herein, the term "polynucleotide" is a DNA or RNA strand having a certain length or more as a polymer of nucleotides in which nucleotide monomers are connected in a long chain by covalent bonds, and more specifically, means a polynucleotide fragment encoding the variant.

The polynucleotide encoding the variant of the present disclosure may include a nucleotide sequence encoding the amino acid sequence represented by SEQ ID NO: 1 or SEQ ID NO: 5. As an example of the present disclosure, the polynucleotide of the present disclosure may have or include a sequence of SEQ ID NO: 2 or SEQ ID NO: 6. Further, the polynucleotide of the present disclosure may consist of or essentially consist of the sequence of SEQ ID NO: 2 or SEQ ID NO: 6.

In the polynucleotide of the present disclosure, various modifications may be made in the coding region as long as the amino acid sequence of the variant of the present disclosure is not changed in consideration of codon degeneracy or codons preferred in organisms that are intended to express the variant of the present disclosure. Specifically, the polynucleotide of the present disclosure may have or include a base sequence having 70% or more, 75% or more, 80% or more, 85% or more, 90% or more, 95% or more, 96% or more, 97% or more, 98% or more, and less than 100% homology or identity to the sequence of SEQ ID NO: 2 or SEQ ID NO: 6, or may consist of or essentially consist of a base sequence having 70% or more, 75% or more, 80% or more, 85% or more, 90% or more, 95% or more, 96% or more, 97% or more, 98% or more, and less than 100% homology or identity to the sequence of SEQ ID NO: 2 or SEQ ID NO: 6, but is not limited thereto. Here, in the sequence having homology or identity, the codon encoding the amino acid corresponding to position 56 of SEQ ID NO: 1 or SEQ ID NO: 5 may be one of the codons encoding alanine, and the codon encoding the amino acid corresponding to position 52 of SEQ ID NO: 5 may be one of the codons encoding valine.

Further, the polynucleotide of the present disclosure may include a probe that may be prepared from a known gene sequence, for example, a sequence without limitation as long as it is a sequence that is able to hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having higher homology or identity, i.e., polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1X SSC, 0.1% SDS, specifically 60°C, 0.1X SSC, 0.1% SDS, and more specifically 68°C, 0.1X SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also include substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tₘ value of 55°C and the above-described conditions. Further, the Tₘ value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook *et al., supra*).

Still another aspect of the present disclosure provides a vector including the polynucleotide of the present disclosure. The vector may be an expression vector for expressing the polynucleotide in a microorganism, but is not limited thereto.

As used herein, the term "vector" may include a DNA construct including a polynucleotide sequence encoding a polypeptide of interest which is operably linked to a suitable expression regulatory region (or expression control sequence) so that the polypeptide of interest may be expressed in a suitable host. The expression regulatory region may include a promoter capable of initiating transcription, any operator sequence for controlling the transcription, a sequence encoding a suitable mRNA ribosome binding site, and a sequence controlling termination of transcription and translation. The vector may be transformed into a suitable microorganism and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, *etc.* may be used as a phage vector or a cosmid vector, and pDZ system, pBR system, pUC system, pBluescript II system, pGEM system, pTZ system, pCL system, pET system, *etc.* may be used as a plasmid vector. Specifically, pDZ, pDC, pDCM2, pACYC177, pACYC184, pCL, pECCG117, pUC19, pBR322, pMW118, pCC1BAC vector, *etc.* may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further include a selection marker for the confirmation of chromosome insertion. The selection marker is for selecting the cells transformed with vectors, i.e., for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector including a polynucleotide encoding a target polypeptide is introduced into a microorganism so that the polypeptide encoded by the polynucleotide may be expressed in the microorganism. The transformed polynucleotide may be located regardless of the position, either by being inserted into the chromosome of the microorganism or located outside the chromosome as long as it may be expressed in the microorganism. The polynucleotide includes DNA and/or RNA encoding a polypeptide of interest. The polynucleotide may be introduced in any form as long as it may be introduced into a microorganism and expressed. For example, the polynucleotide may be introduced into a microorganism in the form of an expression cassette, which is a gene construct containing all elements required for self-expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a microorganism in its own form and operably linked to a sequence required for expression in the microorganism, but is not limited thereto.

Further, the term "operably linked" means that the polynucleotide sequence is functionally linked to a promoter sequence that initiates and mediates transcription of the polynucleotide encoding the variant of interest of the present disclosure.

Still another aspect of the present disclosure provides a microorganism for producing L-branched-chain amino acids, the microorganism including the variant of the present disclosure or the polynucleotide of the present disclosure.

Specifically, the microorganism may be a microorganism of the genus *Corynebacterium,* and more specifically, *Corynebacterium glutamicum,* but is not limited thereto.

The microorganism of the present disclosure may include the modified polypeptide of the present disclosure, the polynucleotide encoding the polypeptide, or the vector including the polynucleotide of the present disclosure.

As used herein, the term "microorganism (or strain)" includes all of wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism including genetic modification for production of a polypeptide, protein, or product of interest.

The strain of the present disclosure may be a strain including any one or more of the variant of the present disclosure, the polynucleotide of the present disclosure, and a vector including the polynucleotide of the present disclosure; a strain modified to express the variant of the present disclosure or the polynucleotide of the present disclosure; a strain (*e.g.,* a recombinant strain) expressing the variant of the present disclosure or the polynucleotide of the present disclosure; or a strain (e.g., a recombinant strain) having the activity of the variant of the present disclosure, but is not limited thereto.

The strain of the present disclosure may be a strain having L-branched-chain amino acid producing ability.

The strain of the present disclosure may be a microorganism naturally having the regulator of acetate metabolism A or L-branched-chain amino acid producing ability, or a microorganism in which the variant of the present disclosure or the polynucleotide encoding the variant (or a vector including the polynucleotide) is introduced into a parent strain that does not have the regulator of acetate metabolism A or L-branched-chain amino acid producing ability and/or in which L-branched-chain amino acid producing ability is conferred on the parent strain, but is not limited thereto.

For example, the strain of the present disclosure is a cell or microorganism that is transformed with the polynucleotide of the present disclosure or a vector including the polynucleotide encoding the variant of the present disclosure to express the variant of the present disclosure. With respect to the objects of the present disclosure, the strain of the present disclosure may include any microorganism that includes the variant of the present disclosure and is able to produce L-branched-chain amino acids. For example, the strain of the present disclosure may be a recombinant strain in which the polynucleotide encoding the variant of the present disclosure is introduced into a natural wild-type microorganism or a microorganism producing L-branched-chain amino acids to express the variant regulator of acetate metabolism A and to have an increased L-branched-chain amino acid producing ability. The recombinant strain having the increased L-branched-chain amino acid producing ability may be a microorganism having an increased L-branched-chain amino acid producing ability, as compared to a natural wild-type microorganism or a microorganism in which the regulator of acetate metabolism A is unmodified (*i.e.*, a microorganism expressing the wild-type regulator of acetate metabolism A (SEQ ID NO: 3) or a microorganism that does not express the modified protein (SEQ ID NO: 1 or SEQ ID NO: 5), but is not limited thereto. For example, the strain having the increased L-branched-chain amino acid producing ability of the present disclosure may be a microorganism having the increased L-branched-chain amino acid producing ability, as compared to a microorganism including the polypeptide of SEQ ID NO: 3 or a polynucleotide encoding the same, but is not limited thereto.

For example, the recombinant strain having the increased producing ability may have an increased L-branched-chain amino acid ability of about 1% or more, specifically, about 1% or more, about 2.5% or more, about 5% or more, about 6% or more, about 7% or more, about 8% or more, about 9% or more, about 10% or more, about 10.5% or more, about 11% or more, about 11.5%or more, about 12% or more, about 12.5% or more, about 13% or more, about 13.5% or more, about 14% or more, about 14.1% or more, about 14.2% or more, about 14.3% or more, about 14.4% or more, about 14.5% or more, or about 14.6% or more (the upper limit is not particularly limited, but may be, for example, about 200% or less, about 150% or less, about 100% or less, about 50% or less, about 45% or less, about 40% or less, about 35% or less, about 30% or less, about 25% or less, about 20% or less, or about 15% or less), as compared to the L-branched-chain amino acid producing ability of the parent strain before being varied or an unmodified microorganism, but the increased amount is not limited thereto as long as the producing ability has an increased amount of a + value, as compared to the producing ability of the parent strain before being varied or an unmodified microorganism. In another example, the recombinant strain having the increased producing ability may have an increased L-branched-chain amino acid producing ability of about 1.1 times or more, about 1.12 times or more, about 1.13 times or more, or 1.14 times or more (the upper limit is not particularly limited, but may be, for example, about 10 times or less, about 5 times or less, about 3 times or less, about 2 times or less, about 1.5 times or less, or about 1.2 times or less), as compared to the L-branched-chain amino acid producing ability of the parent strain before being varied or an unmodified microorganism, but is not limited thereto. The term "about" refers to a range which includes all of ±0.5, ±0.4, ±0.3, ±0.2, ±0.1, *etc.,* and includes all of the values that are equivalent or similar to those following the term "about", but the range is not limited thereto.

As used herein, the term "unmodified microorganism" does not exclude strains including mutation that may occur naturally in microorganisms, and may be a wild-type strain or a natural strain itself or may be a strain before the trait is changed by genetic variation due to natural or artificial factors. For example, the unmodified microorganism may be a strain into which the variant regulator of acetate metabolism A described in the present specification is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

In another example of the present disclosure, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

As used herein, the term "enhancement" of polypeptide activity means that the activity of a polypeptide is increased, as compared to the endogenous activity. The enhancement may be used interchangeably with terms such as activation, up-regulation, overexpression, and increase, *etc.* Here, activation, enhancement, up-regulation, overexpression, and increase may include both exhibiting activity that was not originally possessed and exhibiting improved activity as compared to the endogenous activity or activity before modification. The "endogenous activity" means the activity of a specific polypeptide originally possessed by the parent strain before the trait is changed or an unmodified microorganism when the trait is changed by genetic variation due to natural or artificial factors. This may be used interchangeably with "activity before modification". The fact that the activity of a polypeptide is "enhanced", "up-regulated", "overexpressed", or "increased" as compared to the endogenous activity means that the activity of the polypeptide is improved as compared to the activity and/or concentration (expression level) of a specific polypeptide originally possessed by the parent strain before the trait is changed or an unmodified microorganism.

The enhancement may be achieved through the introduction of a foreign polypeptide or the enhancement of endogenous activity and/or concentration (expression level) of the polypeptide. The enhancement of the activity of a polypeptide may be confirmed by an increase in the degree of activity and the expression level of the corresponding polypeptide or in the amount of the product produced from the corresponding polypeptide.

For the enhancement of the activity of the polypeptide, various methods well known in the art may be applied, and the method is not limited as long as the activity of the polypeptide of interest may be enhanced as compared to that of the microorganism before being modified. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16; Sambrook et al. Molecular Cloning 2012, *etc*.).

Specifically, the enhancement of the polypeptide activity of the present disclosure may be:
1) increase in the intracellular copy number of the polynucleotide encoding the polypeptide;
2) replacement of a gene expression regulatory region on a chromosome encoding the polypeptide with a sequence exhibiting strong activity;
3) modification of a start codon of a gene transcript encoding the polypeptide or a nucleotide sequence encoding a 5'-UTR region;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the polypeptide;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

More specifically:
1) The increase in the intracellular copy number of the polynucleotide encoding the polypeptide may be achieved by the introduction of, into a microorganism, a vector which may replicate and function independently of the host and to which the polynucleotide encoding the corresponding polypeptide is operably linked. Alternatively, the increase may be achieved by the introduction of one copy or two or more copies of the polynucleotide encoding the corresponding polypeptide into a chromosome of a microorganism. The introduction into the chromosome may be performed by introducing a vector capable of inserting the polynucleotide into a chromosome of a microorganism into the microorganism, but is not limited thereto. The vector is as described above.
2) The replacement of a gene expression regulatory region (or expression control sequence) on a chromosome encoding the polypeptide with a sequence exhibiting strong activity may be, for example, occurrence of variation in a sequence due to deletion, insertion, nonconservative or conservative substitution, or a combination thereof, or replacement with a sequence exhibiting stronger activity so that the activity of the expression regulatory region is further enhanced. The expression regulatory region may include, but is not particularly limited to, a promoter, an operator sequence, a sequence encoding a ribosome binding site, a sequence controlling the termination of transcription and translation, *etc.* For example, the replacement may be to replace the original promoter with a strong promoter, but is not limited thereto.
   Examples of known strong promoters include CJ1 to CJ7 promoters (U.S. Patent No. US 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent No. US 10584338 B2), O2 promoter (U.S. Patent No. US 10273491 B2), tkt promoter, yccA promoter, *etc.,* but is not limited thereto.
3) The modification of a start codon of a gene transcript encoding the polypeptide or a nucleotide sequence encoding a 5'-UTR region may be, for example, substitution with a base sequence encoding another start codon having a higher polypeptide expression rate as compared to an endogenous start codon, but is not limited thereto.
4) and 5) The modification of the amino acid sequence or the polynucleotide sequence may be occurrence of variation in the sequence due to deletion, insertion, nonconservative or conservative substitution of the amino acid sequence of the polypeptide or the polynucleotide sequence encoding the polypeptide, or a combination thereof, or replacement with an amino acid sequence or polynucleotide sequence modified to exhibit stronger activity or an amino acid sequence or polynucleotide sequence modified to be more active so that the activity of the polypeptide is enhanced, but is not limited thereto. The replacement may be specifically performed by inserting a polynucleotide into a chromosome by homologous recombination, but is not limited thereto. The vector used here may further include a selection marker for the confirmation of chromosome insertion. The selection marker is as described above.
6) The introduction of a foreign polynucleotide exhibiting the activity of the polypeptide may be introduction of a foreign polynucleotide encoding a polypeptide exhibiting activity identical or similar to that of the polypeptide into a microorganism. There is no limitation in its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in a host cell, the polypeptide may be produced, and the activity thereof may be increased.
7) The codon optimization of the polynucleotide encoding the polypeptide may be codon optimization of an endogenous polynucleotide so as to increase transcription or translation in a microorganism, or codon optimization of a foreign polynucleotide so as to perform optimized transcription and translation in a microorganism.
8) The analysis of the tertiary structure of the polypeptide to select and to modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on this, and to modify or chemically modify the exposed site to be modified or chemically modified.

Such enhancement of the polypeptide activity may be an increase in the activity or concentration expression level of the corresponding polypeptide, based on the activity or concentration of the polypeptide expressed in a wild-type microbial strain or a microbial strain before being modified, or an increase in the amount of a product produced from the corresponding polypeptide, but is not limited thereto.

In the microorganism of the present disclosure, partial or entire modification of the polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation, and/or chemicals, but is not limited thereto. A method of modifying a part or the entirety of the gene may include a method using a DNA recombination technology. For example, by introducing a nucleotide sequence or vector including a nucleotide sequence homologous to the gene of interest into the microorganism to cause homologous recombination, a part or the entirety of the gene may be deleted. The nucleotide sequence or vector to be introduced may include a dominant selection marker, but is not limited thereto.

In the microorganism of the present disclosure, the variant, polynucleotide, L-branched-chain amino acids, *etc.* are as described in other aspects.

Still another aspect of the present disclosure provides a method of producing L-branched-chain amino acids, the method including the step of culturing the microorganism in a medium.

Specifically, the method of producing L-branched-chain amino acids of the present disclosure may include the step of culturing, in a medium, the *Corynebacterium glutamicum* strain including the variant of the present disclosure or the polynucleotide of the present disclosure or the vector of the present disclosure, but is not limited thereto.

As used herein, the term "culturing" means growing the microorganism of the present disclosure under appropriately controlled environmental conditions. The culturing process of the present disclosure may be performed according to suitable medium and culture conditions known in the art. Such a culture process may be easily adjusted and used by those skilled in the art according to the selected strain. Specifically, the culturing may be a batch type, continuous type, and/or fed-batch type, but is not limited thereto.

As used herein, the term "medium" means a mixed substance containing nutrients required to culture the microorganism of the present disclosure as a main component, and the medium supplies nutrients, growth factors, *etc.,* including water, which are indispensable for survival and development. Specifically, as the medium and other culture conditions used for culturing the microorganism of the present disclosure, any one may be used without particular limitation as long as it is a medium used for common culture of microorganisms. The microorganism of the present disclosure may be cultured in a common medium containing proper carbon sources, nitrogen sources, phosphorus sources, inorganic compounds, amino acids and/or vitamins, *etc.,* while controlling the temperature, pH, *etc.* under aerobic conditions.

For example, the culture medium for the strain of the genus *Corynebacterium* may be found in the document ("Manual of Methods for General Bacteriology" by the American Society for Bacteriology (Washington D.C., USA, 1981)).

In the present disclosure, the carbon sources include carbohydrates such as glucose, saccharose, lactose, fructose, sucrose, maltose, *etc*.; sugar alcohols such as mannitol, sorbitol, *etc.,* organic acids such as pyruvic acid, lactic acid, citric acid, *etc*.; amino acids such as glutamic acid, methionine, lysine, *etc.* Natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (i.e., molasses converted to reducing sugar) may be used, and appropriate amounts of other carbon sources may be used in various manners without limitation. These carbon sources may be used singly or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, ammonium nitrate, *etc*.; and organic nitrogen sources such as amino acids such as glutamic acid, methionine, glutamine, *etc.,* peptone, NZ-amine, meat extract, yeast extract, malt extract, corn steep liquor, casein hydrolysate, fish or decomposition products thereof, and skim soybean cake or decomposition products thereof may be used. These nitrogen sources may be used singly or in combination of two or more thereof, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, *etc.* may be used. In addition, amino acids, vitamins and/or suitable precursors, *etc.* may be included. These components or precursors may be added to the medium batchwise or continuously, but is not limited thereto.

Further, during the culturing of the microorganism of the present disclosure, the pH of the medium may be adjusted by adding compounds such as ammonium hydroxide, potassium hydroxide, ammonia, phosphoric acid, sulfuric acid, *etc.* to the medium in a proper manner. Further, during the culturing, foaming may be suppressed by using an antifoaming agent such as fatty acid polyglycol ester. Further, oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but is not limited thereto.

In the culturing of the present disclosure, the culture temperature may be maintained at 20°C to 45°C, specifically at 25°C to 40°C, and the strain may be cultured for about 10 to 160 hours, but are not limited thereto.

The L-branched-chain amino acids produced through the culturing of the present disclosure may be secreted into the medium or may remain in the cells.

The method of producing L-branched-chain amino acids of the present disclosure may further include the steps of preparing the microorganism of the present disclosure, preparing a medium for culturing the strain, or a combination thereof (in any order), for example, prior to the culturing step.

The method of producing L-branched-chain amino acids of the present disclosure may further include the step of recovering L-branched-chain amino acids from the medium according to the culturing (the medium subjected to the culturing) or from the microorganism of the present disclosure. The recovering step may be further included after the culturing step.

The recovering may be to collect L-branched-chain amino acids of interest by way of a suitable method known in the art according to the method of culturing the microorganism of the present disclosure, for example, a batch, continuous, or fed-batch culture method. For example, centrifugation, filtration, treatment with a crystallized protein precipitant (salting out), extraction, sonication, ultrafiltration, dialysis, various forms of chromatography such as molecular sieve chromatography (gel filtration), adsorption chromatography, ion-exchange chromatography, and affinity chromatography, *etc.,* HPLC, or a combination thereof may be used. L-Branched-chain amino acids of interest may be recovered from the medium or microorganism by way of a suitable method known in the art.

The method of producing L-branched-chain amino acids of the present disclosure may further include a purification step. The purification may be performed by way of a suitable method known in the art. In an example, when the method of producing L-branched-chain amino acids of the present disclosure includes both the recovery step and the purification step, the recovery step and the purification step may be performed continuously or discontinuously regardless of the order, or may be performed simultaneously or by being combined into one step, but is not limited thereto.

In the method of the present disclosure, the variant, polynucleotide, vector, microorganism, *etc.* are as described in other aspects.

Still another aspect of the present disclosure provides a composition for producing L-branched-chain amino acids, the composition including the variant of the present disclosure, the polynucleotide encoding the variant, the vector including the polynucleotide, the microorganism including the polynucleotide of the present disclosure; a culture medium thereof; or a combination of two or more thereof.

The composition of the present disclosure may further include arbitrary suitable excipients which are commonly used in compositions for producing amino acids. Such excipients may be, for example, a preservative, a wetting agent, a dispersing agent, a suspending agent, a buffering agent, a stabilizer, or an isotonic agent, *etc.,* but are not limited thereto.

In the composition of the present disclosure, the variant, polynucleotide, vector, strain, medium, L-branched-chain amino acids, *etc.* are as described in other aspects.

Still another aspect of the present disclosure provides use of the variant regulator of acetate metabolism A, in which the amino acid corresponding to position 56 in the amino acid sequence of SEQ ID NO: 3 is substituted with another amino acid, in the production of L-branched-chain amino acids.

In the use of the present disclosure, the variant, L-branched-chain amino acids, *etc.* are as described in other aspects.

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Selection of mutant strains with increased valine producing ability through artificial mutagenesis

### Example 1-1. Artificial mutagenesis through UV irradiation

In order to select mutant strains with increased valine producing ability, *Corynebacterium glutamicum* KCCM11201P (Korean Patent No. 10-1117022), which is a valine-producing strain, was spread on a nutrient medium containing agar and cultured at 30°C for 36 hours. Subsequently, hundreds of colonies thus obtained were irradiated with UV at room temperature to induce random mutations in the genome of the strain.

### Example 1-2. Evaluation of fermentation titer of mutation-induced strain and screening for strain

In order to select mutant strains with increased L-valine producing ability as compared to *Corynebacterium glutamicum* KCCM11201P, which was used as a parent strain, a fermentation titer test was performed on the strains in which random mutations were induced. After each colony was subcultured in a nutrient medium, each strain was inoculated into a 250 mL corner-baffled flask containing 25 mL of a production medium, and cultured at 30°C for 72 hours with shaking at 200 rpm. The compositions of the nutrient medium and production medium are as follows.

### [Nutrient medium (pH 7.2)]

10 g of glucose, 5 g of meat extract, 10 g of polypeptone, 2.5 g of sodium chloride, 5 g of yeast extract, 20 g of agar, 2 g of urea (based on 1 L of distilled water)

### [Production medium (pH 7.0)]

100 g of glucose, 40 g of ammonium sulfate, 2.5 g of soybean protein, 5 g of corn steep solids, 3 g of urea, 1 g of dibasic potassium phosphate, 0.5 g of magnesium sulfate heptahydrate, 100 µg of biotin, 1 mg of thiamine-HCl, 2 mg of calcium pantothenate, 3 mg of nicotinamide, 30 g of calcium carbonate (based on 1 L of distilled water)

After completion of the culture, the production amounts of L-valine were measured using HPLC, and the concentrations of L-valine thus analyzed are shown in Table 1 below.

**[Table 1]**

| | Strain name | L-Valine (g/L) |
|---|---|---|
| Control group | KCCM11201P | 2.8 |
| Experimental group | C1 | 3.1 |
| | C2 | 3.3 |
| | C3 | 2.7 |
| | C4 | 2.1 |
| | C5 | 2.5 |
| | C6 | 1.3 |
| | C7 | 2.8 |
| | C8 | 4.0 |
| | C9 | 3.5 |
| | C10 | 3.2 |
| | C11 | 1.8 |
| | C12 | 2.5 |
| | C13 | 4.4 |
| | C14 | 3.9 |
| | C15 | 3.4 |
| | C16 | 2.1 |
| | C17 | 2.9 |
| | C18 | 2.8 |
| | C19 | 2.6 |
| | C20 | 3.6 |

As shown in Table 1, C13 strain was selected, which showed the highest increase in the valine production, as compared to the control KCCM11201P strain.

### Example 2. Verification of mutation through gene sequencing

Key genes of the C13 strain with the increased valine producing ability were sequenced and compared with those of KCCM1 1201P strain and *Corynebacterium glutamicum* ATCC14067 wild-type strain. As a result, it was confirmed that the C13 strain included nucleotide sequence mutations at specific positions in the open reading frame (ORF) region of ramA gene. Specifically, it was confirmed that the C13 strain had the variant regulator of acetate metabolism A (ramA), in which two mutations were introduced into the base sequence located 166-167 bp downstream from the initiation codon of the ramA gene, resulting in change of the existing CTG into GCG, and substitution of alanine for leucine which is an amino acid at position 56 from the N-terminus.

As a result of analyzing the mutation region, it was confirmed that the effector binding domain of the regulator of acetate metabolism A protein was affected, and it was expected that the activity of the protein would be enhanced.

### Example 3. Preparation of KCCM11201P strain introduced with ramA mutation and examination of valine producing ability

### Example 3-1. Preparation of ramA mutation-introduced strain from Corynebacterium glutamicum KCCM11201P strain and evaluation of L-valine producing ability

In order to insert the ramA(L56A) mutant represented by SEQ ID NO: 1 into *glutamicum* KCCM11201P, a vector containing the target mutation was constructed. In detail, the genomic DNA of the C13 strain was extracted using a G-spin Total DNA Extraction Mini Kit (Intron, Cat. No. 17045) according to the protocol provided in the kit, and PCR was performed using the genomic DNA as a template. PCR was performed under conditions of denaturation at 94°C for 5 minutes; followed by 25 cycles of denaturation at 94°C for 30 seconds, annealing at 55°C for 30 seconds, and polymerization at 72°C for 150 seconds; polymerization at 72°C for 7 minutes, and a PCR product of 1000 bp (hereinafter referred to as "mutation-introduced fragment 1") was obtained using SEQ ID NO: 7 and SEQ ID NO: 8.

Mutation-introduced fragment 1 obtained above was treated with a restriction enzyme Xbal (New England Biolabs, Beverly, MA), and then ligated with a pDZ vector treated with the same restriction enzyme (Korean Patent No. 10-0924065 and International Patent Publication No. 2008-033001) using T4 ligase (New England Biolabs, Beverly, MA). The prepared gene was transformed into *E. coli* DH5α, which was selected in a kanamycin-containing LB medium, and DNA was obtained using a DNA-spin plasmid DNA purification kit (iNtRON) to prepare a vector pDZ-ramA including mutation-introduced fragment 1 (L56A).

**[Table 2]**

| Primer | Nucleotide sequence |
|---|---|
| SEQ ID NO: 7 | ctat tctaga ATGGGGGTAGCGGGTGTG |
| SEQ ID NO: 8 | ctat tctaga GCGGACAGCTTGGTGGT |

Subsequently, in order to further introduce, into *glutamicum* KCCM11201P, ramA(A52V) mutant, in which alanine which is an amino acid at position 52 of the ramA gene related to the known lysine producing ability, was mutated to valine (Metabolic Engineering, Volume 48, 2018, Pages 1-12, ISSN 1096-7176, https://doi.org/10.1016/j.ymben.2018.05.004.), a vector including the target mutation was prepared. In detail, the genomic DNA of the C13 strain was extracted using a G-spin Total DNA Extraction Mini Kit (Intron, Cat. No. 17045) according to the protocol provided in the kit, and PCR was performed using the genomic DNA as a template. PCR was performed under conditions of denaturation at 94°C for 5 minutes; followed by 25 cycles of denaturation at 94°C for 30 seconds, annealing at 55°C for 30 seconds, and polymerization at 72°C for 150 seconds; polymerization at 72°C for 7 minutes, and a PCR product of 502 bp (hereinafter referred to as "mutation-introduced fragment 2") was obtained using SEQ ID NO: 9 and SEQ ID NO: 10, and a PCR product of 500 bp (hereinafter referred to as "mutation-introduced fragment 3") was obtained using SEQ ID NO: 11 and SEQ ID NO: 12.

The mutation-introduced fragments 2 and 3 obtained above were ligated with a pDZ vector treated with Xbal (New England Biolabs, Beverly, MA) (Korean Patent No. 10-0924065 and International Patent Publication No. 2008-033001) using an infusion cloning kit (Takara Bio Inc., Otsu, Japan), and then transformed into *E. coli* DH5α. The prepared gene was transformed into E. *coli* DH5α, and then selected in a kanamycin-containing LB medium, and DNA was obtained using a DNA-spin plasmid DNA purification kit (iNtRON) to prepare a vector pDZ-ramA(A52V+L56A) including mutation-introduced fragments 2 and 3.

**[Table 3]**

| Primer | Nucleotide sequence |
|---|---|
| SEQ ID NO: 9 | cggggatcctctaga CTAATACCGCAAATGG |
| SEQ ID NO: 10 | TGACCAGATTCTGCAGAA |
| SEQ ID NO: 11 | TTCTGCAGAATCTGGTCA |
| SEQ ID NO: 12 | caggtcgactctaga GGTGGTCTGCTTGATG |

Subsequently, the pDZ-ramA(L56A) was transformed into *Corynebacterium glutamicum* KCCM11201P by homologous recombination on the chromosome (van der Rest et al., Appl Microbiol Biotechnol 52:541-545, 1999). The strain in which the vector was inserted onto the chromosome by recombination of the homologous sequence was selected in a medium containing 25 mg/L kanamycin. Then, the *Corynebacterium glutamicum* transformant, in which the secondary recombination was completed, was subjected to PCR using SEQ ID NO: 7 and SEQ ID NO: 8 to identify the strain having substitution of alanine for leucine at position 56 of SEQ ID NO: 3 in the ORF of the ramA gene on the chromosome. The recombinant strain was named *Corynebacterium glutamicum* KCCM11201P::ramA(L56A).

The pDZ-ramA(A52V+L56A) was transformed into *Corynebacterium glutamicum* KCCM11201P by homologous recombination on the chromosome (van der Rest et al., Appl Microbiol Biotechnol 52:541-545, 1999). The strain in which the vector was inserted onto the chromosome by recombination of the homologous sequence was selected in a medium containing 25 mg/L kanamycin. Then, the *Corynebacterium glutamicum* transformant, in which the secondary recombination was completed, was subjected to PCR using SEQ ID NO: 9 and SEQ ID NO: 12 to identify the strain having substitution of valine for alanine at position 52 and substitution of alanine for leucine at position 56 of SEQ ID NO: 3 in the ORF of the ramA gene on the chromosome. The recombinant strain was named *Corynebacterium glutamicum* KCCM11201P::ramA(A52V+L56A).

A flask test was performed to compare the valine producing ability between valine-producing strains, *Corynebacterium glutamicum* KCCM11201P, KCCM11201P::ramA(L56A), and KCCM11201P::ramA(A52V+L56A). Each strain was subcultured in a nutrient medium, and then inoculated into a 250 mL corner-baffled flask containing 25 mL of a production medium, and cultured at 30°C for 72 hours with shaking at 200 rpm, respectively.

### [Nutrient medium (pH 7.2)]

10 g of glucose, 5 g of meat extract, 10 g of polypeptone, 2.5 g of sodium chloride, 5 g of yeast extract, 20 g of agar, 2 g of urea (based on 1 L of distilled water)

### [Production medium (pH 7.0)]

100 g of glucose, 40 g of ammonium sulfate, 2.5 g of soybean protein, 5 g of corn steep solids, 3 g of urea, 1 g of dibasic potassium phosphate, 0.5 g of magnesium sulfate heptahydrate, 100 µg of biotin, 1 mg of thiamine-HCl, 2 mg of calcium pantothenate, 3 mg of nicotinamide, 30 g of calcium carbonate (based on 1 L of distilled water)

After completion of the culture, the L-valine producing ability was measured using HPLC. The concentrations of L-valine thus analyzed are shown in Table 4 below.

**[Table 4]**

| L-Valine producing abilities of KCCM11201P, KCCM11201P::ramA(L56A), and KCCM11201P::ramA(A52V+L56A) strains | | | | |
|---|---|---|---|---|
| Strain | L-Valine (g/L) | | | |
| | Batch 1 | Batch 2 | Batch 3 | Mean |
| KCCM11201P | 2.7 | 2.7 | 2.8 | 2.7 |
| KCCM11201P::*ramA*(L56A) | 3.0 | 3.0 | 3.1 | 3.0 |
| KCCM11201P::*ramA*(A52V+L56A) | 3.1 | 3.2 | 3.1 | 3.1 |

As shown in Table 4, it was confirmed that KCCM11201P::ramA(L56A) and KCCM11201P::ramA(A52V+L56A) strains, into which each variant was introduced, showed 11% and 14.6% increase in the L-valine producing ability, respectively, as compared to the wild-type strain KCCM11201P, which is a parent strain.

### Example 3-2: Preparation of RamA mutation-introduced strain from Corynebacterium glutamicum CJ7V strain and evaluation of L-valine producing ability

In order to examine the effect of increasing L-valine producing ability in strains belonging to other L-valine-producing *Corynebacterium glutamicum,* a kind of mutation (ilvN(A42V); Biotechnology and Bioprocess Engineering, June 2014, Volume 19, Issue 3, pp. 456-467) was introduced into the wild-type *Corynebacterium glutamicum* ATCC14067 to prepare a strain having improved L-valine producing ability.

In detail, the genomic DNA of the wild-type *Corynebacterium glutamicum* ATCC14067 strain was extracted using a G-spin Total DNA Extraction Mini Kit (Intron, Cat. No. 17045) according to the protocol provided in the kit. PCR was performed using the genomic DNA as a template. To construct a vector for introducing the A42V mutation into ilvN gene, gene fragments (A, B) were obtained using a pair of primers of SEQ ID NO: 13 and SEQ ID NO: 14 and a pair of primers of SEQ ID NO: 15 and SEQ ID NO: 16, respectively. PCR was performed under conditions of denaturation at 94°C for 5 minutes; followed by 25 cycles of denaturation at 94°C for 30 seconds, annealing at 55°C for 30 seconds, and polymerization at 72°C for 60 seconds; polymerization at 72°C for 7 minutes.

As a result, both the fragments A and B were obtained as polynucleotides of 537 bp. Overlapping PCR was performed using the two fragments as a template and SEQ ID NO: 13 and SEQ ID NO: 16 to obtain a PCR product of 1044 bp (hereinafter referred to as "mutation-introduced fragment 2").

The mutation-introduced fragment 2 obtained above was treated with a restriction enzyme Xbal (New England Biolabs, Beverly, MA), and then ligated with a pDZ vector treated with the same restriction enzyme using T4 ligase (New England Biolabs, Beverly, MA). The prepared gene was transformed into *E. coli* DH5α, which was selected in a kanamycin-containing LB medium, and DNA was obtained using a DNA-spin plasmid DNA purification kit (iNtRON). The vector for introducing A42V mutation of ilvN gene was named pDZ-ilvN(A42V).

**[Table 5]**

| Primer | Nucleotide sequence |
|---|---|
| SEQ ID NO: 13 | aatttctagaggcagaccctattctatgaagg |
| SEQ ID NO: 14 | agtgtttcggtctttacagacacgagggac |
| SEQ ID NO: 15 | gtccctcgtgtctgtaaagaccgaaacact |
| SEQ ID NO: 16 | aatttctagacgtgggagtgtcactcgcttgg |

Subsequently, the pDZ-ilvN(A42V) was transformed into wild-type *Corynebacterium glutamicum* ATCC14067 by homologous recombination on the chromosome (van der Rest et al., Appl Microbiol Biotechnol 52:541-545, 1999). The strain in which the vector was inserted onto the chromosome by recombination of the homologous sequence was selected in a medium containing 25 mg/L kanamycin. Then, the *Corynebacterium glutamicum* transformant, in which the secondary recombination was completed, was subjected to PCR using SEQ ID NO: 13 and SEQ ID NO: 16 to amplify the gene fragment, and then the mutation-inserted strain was identified by genetic sequence analysis. The recombinant strain was named *Corynebacterium glutamicum* CJ7V.

Subsequently, the *Corynebacterium glutamicum* CJ7V was transformed with each vector in the same manner as in Example 3-1 to prepare strains, which were named *Corynebacterium glutamicum* CJ7V::ramA(L56A), CJ7V::ramA(A52V+L56A), respectively. To compare the L-valine producing ability between the prepared strains, the strains were cultured in the same manner as in Example 3-1, and their L-valine concentrations were analyzed. The L-valine concentrations thus analyzed are shown in Talbr 6 below.

**[Table 6]**

| L-Valine producing abilities of CJ7V, CJ7V::ramA(L56A), and CJ7V:: ramA(A52V+L56A) | | | | |
|---|---|---|---|---|
| Strain | L-Valine (g/L) | | | |
| | Batch 1 | Batch 2 | Batch 3 | Mean |
| *CJ7V* | 3.5 | 3.5 | 3.6 | 3.5 |
| *CJ7V::ramA(L56A)* | 3.8 | 3.9 | 3.8 | 3.8 |
| *CJ7V::ramA(A52V+L56A)* | 3.9 | 4.0 | 4.0 | 4.0 |

As shown in Table 6, it was confirmed that CJ7V::ramA(L56A) and CJ7V::ramA(A52V+L56A) strains, into which each variant was introduced, showed 8.5% and 12.3% increase in the L-valine producing ability, respectively, as compared to CJ7V strain.

### Example 4. Preparation of ramA mutation-introduced strain from Corynebacterium glutamicum KCCM11248P strain having L-isoleucine producing ability and evaluation of L-isoleucine producing ability

In order to examine the effect of increasing L-isoleucine producing ability in a *Corynebacterium glutamicum* strain having L-isoleucine producing ability, a strain was prepared, in which ramA mutation was introduced into a *Corynebacterium glutamicum* L-isoleucine-producing strain KCCM11248P (Korean Patent No. 10-1335789).

In detail, the vector pDZ-ramA(L56A) prepared in Example 3-1 was transformed into *Corynebacterium glutamicum* KCCM11248P by homologous recombination on the chromosome (van der Rest et al., Appl Microbiol Biotechnol 52:541-545, 1999). The strain in which the vector was inserted onto the chromosome by recombination of the homologous sequence was selected in a medium containing 25 mg/L kanamycin. Then, the *Corynebacterium glutamicum* transformant, in which the secondary recombination was completed, was subjected to PCR using SEQ ID NO: 7 and SEQ ID NO: 8 to identify the strain having substitution of alanine for leucine at position 56 of SEQ ID NO: 3 in the ORF of the ramA gene on the chromosome. The recombinant strain was named *Corynebacterium glutamicum* KCCM11248P::ramA(L56A).

The vector pDZ-ramA(A52V+L56A) prepared in Example 3-1 was transformed into *Corynebacterium glutamicum* KCCM11248P by homologous recombination on the chromosome (van der Rest et al., Appl Microbiol Biotechnol 52:541-545, 1999). The strain in which the vector was inserted onto the chromosome by recombination of the homologous sequence was selected in a medium containing 25 mg/L kanamycin. Then, the *Corynebacterium glutamicum* transformant, in which the secondary recombination was completed, was subjected to PCR using SEQ ID NO: 9 and SEQ ID NO: 12 to identify the strain having substitution of valine for alanine at position 52 and substitution of alanine for leucine at position 56 of SEQ ID NO: 3 in the ORF of the ramA gene on the chromosome. The recombinant strain was named *Corynebacterium glutamicum* KCCM11248P::ramA(A52V+L56A).

A flask test was performed to compare the isoleucine producing ability between isoleucine-producing strains, *Corynebacterium glutamicum* KCCM11248P, KCCM11248P::ramA(L56A), and KCCM11248P::ramA(A52V+L56A). Each strain was inoculated into a 250 mL corner-baffled flask containing 25 mL of isoleucine production medium, and cultured at 32°C for 60 hours with shaking at 200 rpm, respectively.

### [Production medium (pH 7.2)]

100 g of glucose, 2 g of yeast extract, 16 g of ammonium sulfate, 1 g of monobasic potassium phosphate, 1 g of magnesium sulfate heptahydrate, 10 mg of iron sulfate heptahydrate, 10 mg of manganese sulfate monohydrate, 200 µg of biotin (based on 1 L of distilled water)

After completion of the culture, the L-isoleucine producing ability was measured using HPLC. The concentrations of L-isoleucine thus analyzed are shown in Table 7 below.

**[Table 7]**

| L-Isoleucine producing abilities of KCCM11248P, KCCM11248P::ramA(L56A), and KCCM11248P::ramA(A52V+L56A) strains | | | | |
|---|---|---|---|---|
| Strain | L-Isoleucine (g/L) | | | |
| | Batch 1 | Batch 2 | Batch 3 | Mean |
| KCCM11248P | 1.4 | 1.5 | 1.5 | 1.5 |
| KCCM11248P::*ramA*(L56A) | 1.5 | 1.6 | 1.7 | 1.6 |
| KCCM11248P::*ramA*(A52V+L56A) | 1.6 | 1.7 | 1.8 | 1.7 |

As shown in Table 7, it was confirmed that KCCM11248P::ramA(L56A) and KCCM11248P::ramA(A52V+L56A) strains, into which each variant was introduced, showed 6.8% and 13.6% increase in the L-isoleucine producing ability, respectively, as compared to the parent strain KCCM11248P.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

### Effect of the invention

When a microorganism including a variant regulator of acetate metabolism A of the present disclosure is cultured, it is possible to produce L-branched-chain amino acids with high yield, as compared to an existing microorganism having an unmodified polypeptide.

## Claims

1. A variant regulator of acetate metabolism A, wherein an amino acid corresponding to position 56 in an amino acid sequence of SEQ ID NO: 3 is substituted with another amino acid.

2. The variant of claim 1, wherein an amino acid corresponding to position 52 in the amino acid sequence of SEQ ID NO: 3 is further substituted with another amino acid.

3. The variant of claim 1, wherein the amino acid corresponding to position 56 of SEQ ID NO: 3 is substituted with alanine.

4. The variant of claim 2, wherein the amino acid corresponding to position 56 and the amino acid corresponding to position 52 of SEQ ID NO: 3 are substituted with alanine and valine, respectively.

5. The variant of claim 1, wherein the variant consists of an amino acid sequence represented by SEQ ID NO: 1.

6. The variant of claim 2, wherein the variant consists of an amino acid sequence represented by SEQ ID NO: 5.

7. A polynucleotide encoding the variant of any one of claims 1 to 6.

8. A microorganism for producing L-branched-chain amino acids, the microorganism comprising the variant of any one of claims 1 to 6 or a polynucleotide encoding the variant.

9. The microorganism for producing L-branched-chain amino acids of claim 8, wherein the microorganism has an increased L-branched-chain amino acid producing ability, as compared to a microorganism including a polypeptide of SEQ ID NO: 3 or a polynucleotide encoding the same.

10. The microorganism for producing L-branched-chain amino acids of claim 8, wherein the microorganism is a microorganism of the genus *Corynebacterium.*

11. The microorganism for producing L-branched-chain amino acids of claim 10, wherein the microorganism of the genus *Corynebacterium* is *Corynebacterium glutamicum.*

12. A method of producing L-branched-chain amino acids, the method comprising the step of culturing the microorganism of claim 8 in a medium.

13. The method of claim 12, further comprising the step of recovering a target substance from the medium.
